# EUROPEAN PATENT APPLICATION

(11) **EP 4 711 358 A1**
(43) Date of publication of application: **18.03.2026**
(21) Application number: 24200658.3
(22) Date of filing: 16.09.2024
(51) Int. Cl.: C07D 251/60, C07D 251/62

(54) **MELAMINE PRODUCTION PROCESS FROM PYROLYSIS OF UREA**

(71) Applicant: CASALE SA, 6900 Lugano (CH)
(72) Inventor: GAMBA, Simone, 24040 Pagazzano (BG) (IT); DI CARLO, Gabriele, 6900 Lugano (CH); SCOTTO, Andrea, 6932 Breganzona (CH)
(74) Representative: M. Zardi & Co S.A.

(57) **Abstract**

A high-pressure melamine production process wherein raw melamine is processed including that solid melamine is separated from a mother liquor (1) comprising water, ammonia, carbon dioxide, residual melamine and OAT; said mother liquor is processed including removal of ammonia (100) and treatment of the ammonia-depleted liquor (4) in an OAT decomposition section (200); a purification treatment (300) of the OAT-depleted stream (6) produces a vapour stream (7) containing ammonia, CO2, and water, a first liquid stream (8) and a second liquid stream (9); said first liquid stream (8) contains residual ammonia and has a concentration of CO2 not greater than a target concentration; said second liquid stream (9) has a concentration of ammonia not greater than a target concentration; at least part of said first liquid stream and/or at least part of said second liquid stream is recycled to the melamine production process.

## Description

### Field of application

The invention is in the field of industrial production of high-purity melamine from urea. The invention particularly relates to a process for the melamine purification using ammonia as basic aid, the process further including the treatment of an aqueous effluent purged from said melamine purification for ammonia and carbon dioxide recovery.

### Prior art

Melamine is produced at an industrial scale starting from urea following either a non-catalytic high-pressure (HP) process or a low-pressure (LP) catalytic process. The non-catalytic high-pressure process is considered the most advantageous and is becoming predominant. In the high-pressure process, a urea melt is reacted under suitable melamine-forming conditions; the pressure is generally above 7 MPa, typically 7 to 20 MPa, and the temperature is typically around 375 °C.

The output of the melamine synthesis section is a melamine melt which is sent to a purification section. The synthesis section may include a single reactor or two reactors arranged in series. In an embodiment with two reactors, a melamine stream produced in the first reactor is further processed in the second reactor to remove carbon dioxide using a stripping agent, such as ammonia, obtaining the above-said melamine melt. This second reactor may be termed post-reactor or stripping reactor. The first reactor and the stripping reactor may be two separate vessels or combined in a single apparatus.

The reaction of urea to melamine produces a gaseous stream termed melamine offgas, which contains mainly ammonia and carbon dioxide, together with some melamine and other minor components. Said offgas is usually recycled as a feed material for a tied-in urea plant, after washing in a scrubber to recover the melamine contained therein. The offgas washing may be performed with urea melt or water (aqueous washing).

A combination of production of urea and melamine is attractive because urea is the reagent for the production of melamine, and the offgas released during the synthesis of melamine contains ammonia and carbon dioxide which are the starting materials for the synthesis of urea.

The melamine melt produced by the melamine synthesis section is usually treated in a purification section operating at lower pressure than the melamine synthesis section. Said purification section produces solid melamine with a desired purity.

The processing of the melamine melt includes, typically, the following steps. The melt is firstly quenched and dissolved in a solution of ammonia or sodium hydroxide forming a basic solution. Such an alkaline environment is required for carrying out the following purification steps. A suitable contact time is provided to the basic solution of melamine in order to decompose polycondensates. The resulting solution typically passes through a filtration step and then through a crystallization step wherein solid crystals of melamine are formed. Finally, solid melamine is separated from a liquid stream, termed mother liquor, in a solid-liquid separation step. The solid melamine, that can be washed with water, is dried with hot air before being withdrawn from the process, whereas the aqueous stream is in part recycled to the purification section and in part sent to a suitable treatment section to recover ammonia, remove carbon dioxide and decompose melamine and oxyaminotriazines (OAT).

In case the alkaline agent used in the step of quenching is ammonia, the treatment of said aqueous stream (mother liquor) has three main objectives. Firstly, ammonia contained in the aqueous stream is recovered and recycled in the purification section as basic agent, to provide the required alkaline environment. A second aim is the elimination of the OATs to prevent their accumulation in the melamine purification section and particularly in the crystallization step. Another aim is the removal of carbon dioxide to avoid the lowering of pH in the melamine purification section, specifically in the crystallization step.

The processing of the mother liquor is a considerable source of cost in terms of capital cost of the equipment and energy consumption.

EP 4 245 755 A1 discloses that the mother liquor is treated producing: an ammonia solution to be recycled as a basic agent for the melamine purification, a gaseous stream produced by thermal decomposition of OAT and melamine, and a purified liquid water stream which is recycled in the melamine purification section, in particular for mixing the basic agent (ammonia) with raw melamine. Such process can be improved in terms of reduced duty required by said treatment section.

### Summary of the invention

The invention addresses the problem of how to improve the processing of the mother liquor in a high-pressure melamine synthesis process wherein the melamine melt from the melamine synthesis section is purified using ammonia as basic agent. More in detail, the invention addresses the problem of how to reduce the consumption of energy in the processing of the mother liquor.

The problem is solved with a process according to the claims.

In the present invention, the processing of the mother liquor includes: removal of ammonia, producing an ammonia-depleted aqueous stream; treatment of said ammonia-depleted aqueous stream in an OAT decomposition section where OATs are decomposed forming a OAT-depleted aqueous stream; a purification treatment of said OAT-depleted aqueous stream, thereinafter said purification treatment is also named water purification treatment.

The processing of the mother liquor further includes that said water purification treatment is performed to produce: a vapour stream containing ammonia, CO2, and water; a first liquid stream containing residual ammonia and CO2 with concentration not greater than a target CO2 concentration; a second liquid stream containing ammonia at a concentration not greater than a target ammonia concentration.

The invention is based on the judicious insight of processing the aqueous stream, after removal of OAT, obtaining two separate liquid streams with a different composition, particularly with different requirements in terms of ammonia content. The invention goes against the common approach of pushing the removal of ammonia from the entire aqueous stream, thanks to the understanding that a liquid stream containing ammonia can also be recycled appropriately to the melamine process, provided that such stream has a low content of CO2.

At least part of the first liquid stream and/or at least part of the second liquid stream is recycled to the melamine production process. The recycle is made preferably to the raw melamine purification process. In particular, the ammonia contained in said first liquid stream provides at least part of the basicity required to the steps of purification of said raw melamine.

The above-mentioned target CO2 concentration and target ammonia concentration are the maximum concentrations allowed in the liquid streams and may be selected in accordance with the use of the recycled liquid streams. The target CO2 concentration is preferably 500 ppm by weight, more preferably 250 ppm by weight, even more preferably 150 ppm by weight. The target ammonia concentration is preferably 50 ppm by weight, more preferably 25 ppm by weight, even more preferably 10 ppm by weight.

The invention comes also from the unexpected finding that the water purification treatment requires less heat input when an additional liquid stream containing ammonia (first liquid stream) is produced, in addition to a second liquid stream with a controlled ammonia content, instead of performing the purification treatment to produce a single liquid stream. The first liquid stream, which may have a relatively high content of ammonia, can be recycled for example as basic agent in the raw melamine purification section, leading to a lower energy consumption. Accordingly, the invention allows to carry out a milder (less energy consuming) purification treatment, because a very low content of ammonia is required only for the second liquid stream generated in the treatment process of the mother liquor.

### Description of the invention

The invention concerns a high-pressure process for the production of melamine wherein a stream of melamine melt, produced in a melamine synthesis section, is subject to a purification process including a quenching step performed by mixing the melamine melt with ammonia or with an ammonia solution, resulting in a quenched melamine stream (ammonia solution of melamine) with an increased pH required for the following purification and crystallization steps.

Said quenched melamine stream is subject to crystallization of melamine and subsequent solid-liquid separation, said solid-liquid separation resulting in solid melamine separated from a mother liquor which is an aqueous stream comprising water, ammonia, carbon dioxide, residual melamine and OAT.

Said mother liquor is processed including: removal of ammonia, producing an ammonia-depleted aqueous stream; treatment of said ammonia-depleted aqueous stream in an OAT decomposition section where OAT are decomposed forming a OAT-depleted aqueous stream; a purification treatment of said OAT-depleted aqueous stream (water purification treatment).

Preferably, the water purification treatment is performed in at least one stripping column.

Said water purification treatment produces three streams: a first liquid stream containing residual ammonia and CO2 at a concentration not greater than a target CO2 concentration, a second liquid stream comprising ammonia at a concentration not greater than a target ammonia concentration and a vapour stream containing ammonia, CO2, and water. In an embodiment, said first liquid stream is withdrawn as a lateral stream of said stripping column, said second liquid stream is a bottom stream of said stripping column and the vapour stream is withdrawn from the top of said column.

In a preferred embodiment of the process of the invention, at least part of said first liquid stream and/or at least part of the second liquid stream are recycled in the process. Particularly, the first liquid stream is preferably used to directly generate the ammonia solution to be mixed with the raw melamine stream forming the quenched melamine stream. The ammonia contained in said first liquid stream contributes to the formation of the required basic environment.

At least part of the second liquid stream is preferably recycled to scrub a stream of exhaust air and other ammonia-containing process vents, wherein said stream of exhaust air is withdrawn from a dryer used to dry the solid melamine effluent from the solid-liquid separation and the ammonia-containing process vents are withdrawn from different sections of the plant and washed with water. The aim of the scrubbing system is to obtain a washed process vent with an ammonia content within environmental limits to be discharged to the atmosphere. For such a reason, is of a paramount importance that the vent washing medium is free or substantially free from ammonia. Therefore, the said second liquid stream from the water purification treatment is particularly suitable for this aim. In addition, said second liquid stream may be, at least in part, recycled as a water make-up to the melamine purification process. In a preferred embodiment, said second liquid stream after ammonia absorption in the vent scrubbing section is recycled to melamine purification process.

The removal of ammonia from the mother liquor can be performed in an ammonia removal section. In an interesting embodiment, the vapour stream obtained from the purification treatment of the OAT-depleted aqueous stream is sent to said ammonia removal section to recover ammonia and CO2 contained therein. Preferably, the mother liquor is heated up to a temperature from 70 °C to 95 °C before being treated for ammonia removal.

In a further preferred embodiment, the water purification treatment is performed at a pressure greater than the pressure of the ammonia removal section.

In an embodiment, the vapour stream effluent from the top of said stripping column of the water purification treatment is subjected to total condensation in a total condenser and the resulting condensed stream is in part recycled as reflux to the water purification treatment itself and/or in part preferably recycled to a tied-in urea production plant. Said condensed stream may be sent as such to the tied-in urea production plant, or may be used to facilitate condensation of melamine offgas in an offgas condensation step before being recycled to the tied-in urea plant. The expression "total condensation" is intended to mean that substantially all the vapour stream fed to the total condenser is condensed, resulting in a liquid effluent. A small amount of residual vapour may remain in the condenser effluent.

In the embodiment with the above-mentioned total condensation, the water content of the liquid product from the top of the water purification treatment, thanks to the reflux, is lower than the water content of the vapour stream obtained when the water purification treatment does not make use of a total condenser. Therefore, in such embodiment, there is no longer the need to recycle the top product of the water purification treatment to the ammonia removal and said liquid product can be recycled to a tied-in urea production plant directly or via the offgas condensation.

Preferably, the water purification treatment is operated at a pressure from 2 barg to 7 barg, more preferably from 2.5 barg to 5 barg. In the description and claims, the pressure is given in bar gauge.

In a still preferred embodiment, the ammonia removal step is performed in a distillation column. Particularly preferably, said distillation column is provided with a partial condenser which is connected to the column to receive a stream of overhead vapour from top of the column.

Said distillation column receives a heat input from a suitable heat source, such as hot steam and/or a reboiler, to provide the heat required by said ammonia removal step.

The ammonia-depleted aqueous stream can be withdrawn from the bottom of said distillation column and sent to a treatment to decompose melamine and OATs in an OAT decomposition section. In an embodiment, said ammonia-depleted aqueous stream has a concentration of ammonia not higher than 5000 ppm by weight, preferably not higher than 2500 ppm by weight, more preferably not higher than 1000 ppm by weight.

In a further embodiment, an effluent from the top of said distillation column is partially condensed in said partial condenser, to form an ammonia-rich vapour stream that can be recycled to the process and an aqueous stream containing ammonium carbonate.

Said ammonia-rich vapour stream comprises ammonia, water and traces of carbon dioxide. Preferably said vapour stream has a concentration of ammonia of at least 80% by weight, more preferably at least 85% by weight, still more preferably at least 90%; also preferably, said vapour stream has a concentration of CO2 not greater than 1.0% by weight, more preferably not greater than 0.6% by weight.

Said aqueous stream containing ammonium carbonate allows purge of carbon dioxide as ammonium carbonate from the process, being substantially in liquid/vapour equilibrium with the ammonia-rich vapour stream and comprising ammonia, carbon dioxide and water. Said aqueous stream may be the effluent of said partial condenser of the distillation column. In a preferred embodiment, the mass fraction of water of said liquid stream is less than 75%, preferably from 40% to 75% by weight, more preferably from 45% to 70% by weight, still more preferably from 50% to 65% by weight.

Said ammonia rich-vapour stream may be recycled to the raw melamine purification section, preferably together with the first liquid stream effluent from the stripping column, to be mixed with said raw melamine stream to form the quenched melamine stream. Said aqueous stream containing ammonium carbonate may be sent to a tied-in urea plant, preferably after mixing with a stream of anhydrous offgas produced in the melamine synthesis section. More preferably said stream of anhydrous offgas is scrubbed with urea before mixing with said aqueous stream.

Preferably, the ammonia removal is operated at a pressure that ranges from 0.5 barg to 5 barg, more preferably from 1 barg to 3 barg. Said operating pressure should be high enough to have a temperature at the partial condenser so that cooling water may be used as cooling medium, but also low enough to allow a suitable production of the ammonia-rich vapour stream.

Preferably, said partial condenser is operated at a temperature from 50 °C to 70 °C, more preferably from 55 °C to 65 °C.

In an embodiment, at least 70%, preferably at least 80%, of the ammonia fed in the distillation column is withdrawn from said column in the ammonia-rich vapour stream. In a further embodiment, at least 85%, preferably at least 90%, of the carbon dioxide fed in the distillation column is withdrawn from said column in said aqueous stream containing ammonium carbonate.

In a still further preferred embodiment, the OAT decomposition section operates at a temperature of at least 230 °C, preferably at least 250 °C, more preferably at least 270 °C, still more preferably at least 285 °C, and at a pressure greater than the vapour pressure of water at the temperature of operation of said decomposition section.

In an embodiment, the OAT decomposition section produces a vapour stream that is: sent to condensation of offgas and then recycled in a tied-in urea production process, and/or sent to the above-mentioned distillation column, and/or sent to the water purification treatment.

The OAT-depleted aqueous stream has a concentration of melamine preferably not higher than 500 ppm by weight, more preferably not higher than 100 ppm by weight, still more preferably not higher than 20 ppm by weight.

### Brief description of the figures

Fig. 1 shows a block scheme of a process for treating a mother liquor effluent from a raw melamine purification section, according to an embodiment of the invention.
Fig. 2 illustrates a preferred embodiment of a distillation column for ammonia removal from an aqueous stream derived from a raw melamine stream.
Fig. 3 illustrates a preferred embodiment of a water purification treatment.
Fig. 4 shows a diagram of a process for the production of melamine according to an embodiment of the invention.

### Detailed description of the figures

After melamine synthesis at high pressure (7 to 20 MPa) and high temperature (typically around 375 °C), the resulting raw liquid melamine stream is treated in a melamine purification section wherein a high-purity (not less than 99.6% by weight, preferably not less than 99.8% by weight) solid melamine is produced, and an aqueous stream 1 (mother liquor) is purged. Said stream 1 contains mainly water, ammonia, carbon dioxide, melamine, and OAT.

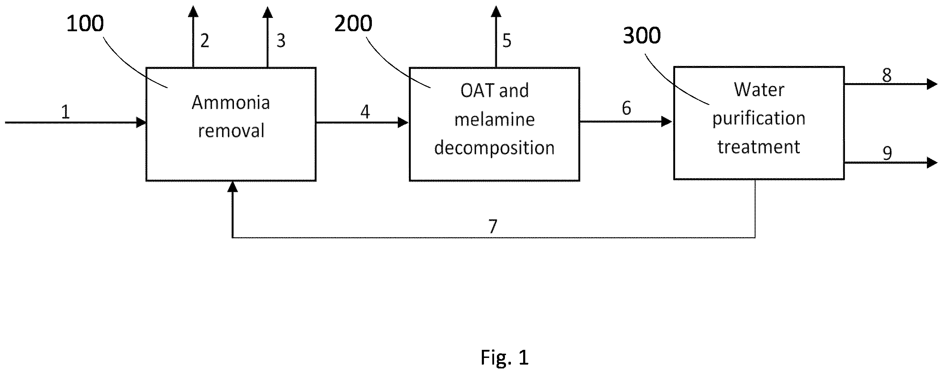

Said aqueous stream 1 (Fig. 1) is firstly introduced in an ammonia removal section 100 producing: an ammonia-rich vapour stream 2 comprising ammonia, water and traces of carbon dioxide; a liquid stream 3 comprising water, ammonia and carbon dioxide; an ammonia-depleted aqueous stream 4.

The liquid stream 3 allows purge of carbon dioxide as ammonium carbonate from the melamine production process, being substantially in liquid/vapour equilibrium with the vapour stream 2. The ammonia-depleted aqueous stream 4 contains residual melamine and OAT and is substantially free of ammonia and carbon dioxide.

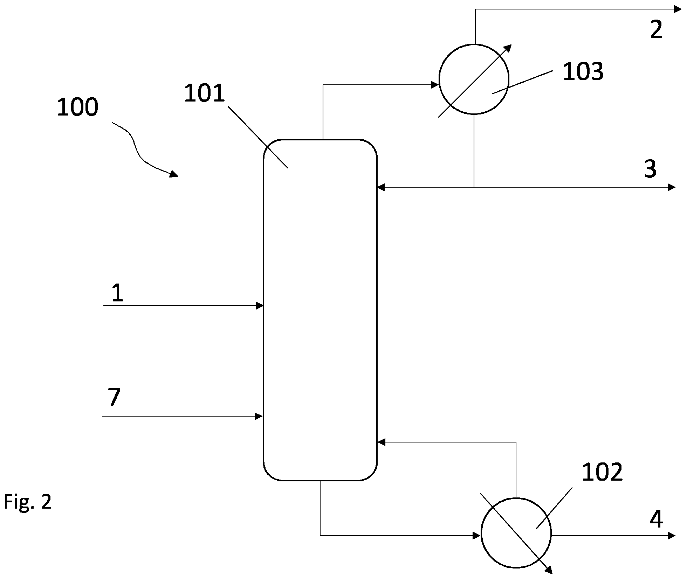

Fig. 2 is a schematic representation of the ammonia removal section 100 including a distillation column 101 connected to a partial condenser 103 and to a reboiler 102. Effluents of the partial condenser 103 are the ammonia-rich vapour stream 2 and the liquid stream 3, as above described. The ammonia-depleted aqueous stream 4 is withdrawn from the bottom of the column 101.

Said aqueous stream 4 is sent to the OAT/melamine decomposition section 200 wherein OAT and melamine are thermally decomposed to CO2 and NH3 via hydrolysis, resulting in a vapour stream 5, comprising water, NH3 and CO2 and an OAT-depleted aqueous stream 6.

The OAT-depleted aqueous stream 6 is then subjected to a water purification treatment in the section 300 wherein said aqueous stream 6 is stripped to produce a vapour stream 7, which is recycled in the ammonia removal unit 100 to recover NH3 and CO2 contained therein, and two liquid streams 8 and 9.

The water purification section 300 is arranged so that the first liquid stream 8 contains some ammonia but has a concentration of CO2 below a selected threshold, and the second liquid stream 9 has a lower concentration of ammonia than said stream 8, the ammonia concentration in said second stream 9 being below a selected threshold. These two liquid streams 8, 9 may be re-utilized in melamine plant, preferably in two different sections according to their different composition.

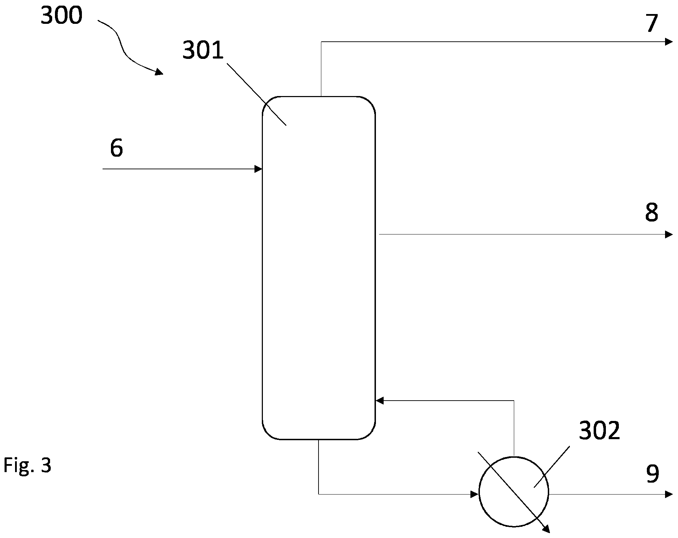

Fig. 3 illustrates an embodiment wherein the water purification section 300 includes a stripping column 301 heated by a bottom reboiler 302. In another embodiment the stripping column 301 may have live steam injection or a combination of live steam and reboiler. The liquid stream 9 is withdrawn from the bottom of the column 301, whereas the liquid stream 8 is a lateral fraction of said column 301.

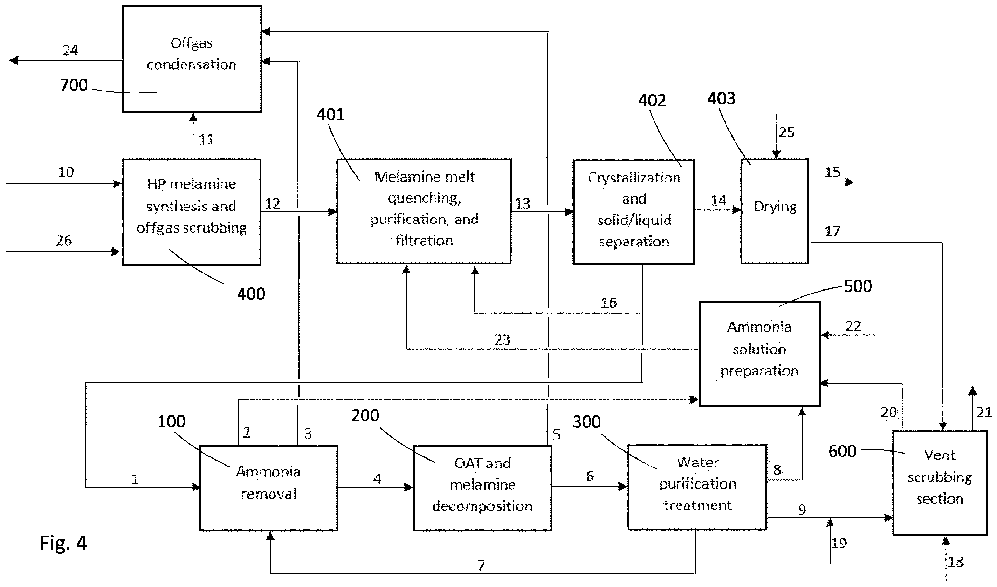

Fig. 4 illustrates a preferred embodiment of the invention in a greater detail.

A urea melt 10 is reacted at high pressure (7 to 20 MPa) and high temperature (around 375 °C) in presence of ammonia 26, producing a raw melamine melt 12 and an offgas 11 comprising NH3 and CO2 that is scrubbed with urea, preferably with the urea melt 10. Said raw melamine melt 12 is treated in a melamine purification section wherein a high-purity solid melamine 15 is produced, and the aqueous stream 1 (mother liquor) is purged.

Said melamine purification section comprises a first step 401 wherein the raw melamine stream 12 is quenched with a stream of ammonia solution 23 and a stream of recycled mother liquor 16, hydrolysed to remove polycondensates and filtered. The resulting purified and filtered melamine stream 13 is subject to melamine crystallization and separation 402, producing a melamine cake 14 that is dried 403 with drying air 25, producing the solid melamine 15 and a stream of exhaust air 17 to be scrubbed. A portion 16 of the mother liquor effluent from the separation 402 is recycled back to the first step 401.

The mother liquor 1 purged from said melamine purification section is sent to the above-described (Fig. 1) sections of ammonia removal 100, OAT decomposition 200 and water purification section 300. Effluents from said sections 100, 200, 300 are the vapour streams 2, 5 and the liquid streams 3, 8 and 9.

The vapour stream 2 is recycled to an ammonia solution preparation section 500 where said stream 2 is condensed by absorption in the first liquid stream 8 and in an ammonia solution 20 coming from a scrubbing section; an additional stream, the ammonia make-up stream 22, is fed to section 500. The resulting ammonia solution 23 effluent from said preparation section 500 is recycled as basic agent to the step of quenching 401.

The streams 5 and 3 are joined with the offgas 11 effluent from the melamine synthesis section 400, condensed 700 and recycled, as the stream 24, to a tied-in urea production plant.

The second liquid stream 9, having a low content of ammonia (e.g. below 10 ppm by weight), may be discharged if necessary or may be recycled, together with a make-up water 19, to a scrubbing section 600. Said scrubbing section 600 treats the exhaust drying air 17 and other ammonia-containing vents 18 from the plant, producing an ammonia-free or substantially free vent 21 that can be released in atmosphere. The scrubbing section further produces the ammonia solution 20 recycled to the ammonia solution preparation section 500.

### Examples

In the following examples, the compositions are given in % or ppm by weight.

### Example 1 - invention

The ammonia removal section (100) receives 1000.0 kg/h of an aqueous stream (1) having the following composition: 14% of NH3, 0.2% of CO2, 0.8% of melamine, 0.25% of OAT and 84.75% of water.

A flow rate of 74.9 kg/h of a vapour stream (7) effluent from the water purification treatment (300) is recycled to the ammonia removal section (100), the stream (7) having the following composition: 6.29% of NH3, 10.46% of CO2 and 83.25% of water.

Thus, a total of 144.7 kg/h of NH3 and 9.83 kg/h of CO2 are fed to said ammonia removal section (100), which is operated at 2 barg and with a condenser (103) temperature of 60 °C.

The ammonia removal section (100) produces:
- 121.5 kg/h of an ammonia-rich vapour stream (2) effluent from the top of the column (101) having the following composition: 95.75% of NH3, 0.39% of CO2, 3.86% of water. Thus, this stream (2) contains 116.3 kg/h of NH3 which is the 80.4% of the total ammonia fed to the column (101);
- 101.6 kg/h of a liquid stream (3) effluent from the top of the column (101) having the following composition: 27.79% of NH3, 9.21% of CO2, 63.00% of water. Thus, this stream (3) contains 9.36 kg/h of CO2 which is the 95.2% of the total CO2 fed to the column (101);
- 851.8 kg/h of an ammonia-depleted aqueous stream (4) having the following composition: 200 ppm_{wt} of NH3, 0.94% of melamine, 0.29% of OAT and 98.75% of water. This stream is substantially free of CO2 (< 1 ppm_{wt}).

The ammonia-depleted aqueous stream (4) is fed to the OAT/melamine decomposition section (200) that operates at 288 °C and 95 barg, producing:
- 9.1 kg/h of a vapour stream (5) comprising 1.82% of NH3, 33.76% of CO2 and 64.42% of water;
- 842.7 kg/h of OAT-depleted aqueous stream (6) comprising 0.97% of NH3, 0.94% of CO2, 10 ppm_{wt} of melamine, 98.09% of water and less than 1 ppm_{wt} of OAT.

Stream (6) has a CO2 content sensibly higher than stream (1), therefore its direct recycle to the melamine purification process prior to the removal of the CO2 has to be avoided in order to control the pH required for melamine purification and crystallization.

The OAT-depleted aqueous stream (6) is sent to the water purification treatment section (300) which operates at 4 barg.

In addition to the vapour stream (7), this section (300) produces:
- 573.0 kg/h of a first liquid stream (8) comprising 0.60% of NH3, 100 ppm_{wt} of CO2, 10.3 ppm_{wt} of melamine, 99.39% of water and less than 1 ppm_{wt} of OAT;
- 194.8 kg/h of a second stream (9) comprising: 1 ppm_{wt} of NH3, less than 1 ppm_{wt} of CO2 and OAT, 13 ppm_{wt} melamine and the remaining part is water.

The ammonia removal section (100) requires a duty of 126.5 kW, while the water purification section (300) requires a duty of 31.0 kW. The total duty is therefore 157.5 kW.

The bottom temperature of the ammonia removal column (101) is 133.7 °C, while the bottom temperature of the stripping column (301) of the water purification is 152 °C. The duty of the water purification treatment section (300) must therefore be supplied at a higher thermal level than that required by the ammonia removal section (100). Therefore, configurations in which most of the duty must be supplied to the ammonia removal section (100) than to the water purification section (300) are preferable.

In addition, the total water sent to a tied-in urea plant via streams 3 and 5 is 69.9 kg/h.

### Example 2 - conventional process

The configuration of this example is the same as the configuration of the previous one, except that the water purification treatment (300) produces only one liquid stream (9) instead of two liquid streams (8, 9).

The ammonia removal section (100) receives the same aqueous stream (1) of the example above, having the same flow rate and composition. A flow rate of 187.3 kg/h of a vapour stream (7) effluent from the water purification treatment (300) is recycled to the ammonia removal section (100), the stream (7) having the following composition: 4.40% of NH3, 4.70% of CO2 and 90.90% of water.

Thus, a total of 148.2 kg/h of NH3 and 10.8 kg/h of CO2 are fed to said ammonia removal section (100), that is operated at 2 barg and with a condenser (103) temperature of 60°C.

The ammonia removal section (100) produces:
- 122.1 kg/h of an ammonia-rich vapour stream (2) effluent from the top of the column (101) having the following composition: 95.75% of NH3, 0.39% of CO2, 3.86% of water. Thus, this stream (2) contains 116.9 kg/h of NH3 which is the 78.9% of the total ammonia fed to the column (101);
- 112.0 kg/h of a liquid stream (3) effluent from the top of the column (101) having the following composition: 27.79% of NH3, 9.21% of CO2, 63.00% of water. Thus, this stream (3) contains 10.3 kg/h of CO2 which is the 95.4% of the total CO2 fed to the column (101);
- 953.2 kg/h of an ammonia-depleted aqueous stream (4) having the following composition: 200 ppm_{wt} of NH3, 0.84% of melamine, 0.26% of OAT and 98.88% of water. This stream is substantially free of CO2 (< 1 ppm_{wt}).

The ammonia-depleted aqueous stream (4) is fed to the OAT/melamine decomposition section (200) that operates at 288 °C and 95 barg, producing:
- 6.4 kg/h of a vapour stream (5) comprising 1.63% of NH3, 34.01% of CO2 and 64.36% of water;
- 946.8 kg/h of OAT-depleted aqueous stream (6) comprising 0.87% of NH3, 0.93% of CO2, 10 ppm_{wt} of melamine, 98.20% of water and less than 1 ppm_{wt} of OAT.

Stream (6) has a CO2 content sensibly higher than stream (1), therefore its direct recycle to the melamine purification process prior to the removal of the CO2 has to be avoided in order to control the pH required for melamine purification and crystallization.

The OAT-depleted aqueous stream (6) is sent to the water purification treatment section (300) which operates at 4 barg.

In addition to the vapour stream (7), this section (300) produces 759.5 kg/h of a liquid stream (9) comprising: 1 ppm_{wt} of NH3, less than 1 ppm_{wt} of CO2 and OAT, 12.5 ppm_{wt} melamine and the remaining part is water.

The ammonia removal section (100) requires a duty of 108.1 kW, while the water purification section (300) requires a duty of 96.9 kW. The total duty is therefore 205 kW.

The bottom temperature of the ammonia removal column (101) is 133.7°C, while the bottom temperature of the stripping column (301) of the water purification is 152°C. The duty of the water purification treatment section (300) must therefore be supplied at a higher thermal level than that required by the ammonia removal section (100). In this case, however, the duty to be supplied is more unbalanced toward the water purification treatment section (300).

In addition, the total water sent to a tied-in urea plant (via streams 3 and 5) is 74.7 kg/h.

### Considerations about examples

A comparison of the examples makes it clear that for substantially the same recovery of ammonia and carbon dioxide fed to the ammonia removal (100), respectively in the vapour stream (2) and in the liquid stream (3), the process of the invention (Example 1), with respect to the conventional process of Example 2, provides:
- a saving of 23% of the total duty required;
- the delivery of a lower flow rate of water to a tied-in urea plant, about 94% of a conventional process;
- a duty to be supplied in a larger quantity at a lower thermal level, i.e., in a higher percentage to the ammonia removal section (100) and in a lower percentage to the water purification section (300). As a matter of facts, according to the embodiment of the invention described in Example 1, 80.3% of the total duty is supplied to ammonia removal section (100), while according to the conventional process of the Example 2, only 52.7% of the total duty is supplied to said section (100).

In addition, the conventional process, that does not produce the first liquid stream (8), implies a higher flow rate of ammonia-depleted aqueous stream (4) that subsequently causes a higher load to the melamine/OAT decomposition section (200).

Furthermore, the conventional process has a lower (< 80%) recovery of ammonia in the ammonia removal section (100) with respect to the process of the invention, despite the higher total energy consumption per unit of aqueous stream (1) treated.

The advantages of the process of the invention are due to the presence of the liquid stream (8) and thus remain even in the case of embodiments wherein the vapour stream (5) is not present, or in embodiments wherein the vapour stream (7) is not recycled to the ammonia removal section (100) but is sent to a tied-in urea plant.

## Claims

1. A process for the production of melamine including:
Urea (10) is reacted according to a non-catalytic high-pressure melamine synthesis process (400), producing an offgas stream (11) comprising ammonia and carbon dioxide and a melamine melt (12);
said melamine melt is subject to a purification process including: quenching (401) of the melamine melt (12) by mixing with ammonia or with an ammonia solution (23), resulting in a quenched stream (13); said quenched stream is subjected at least to steps of crystallization of melamine and subsequent solid-liquid separation (402), said solid-liquid separation resulting in solid melamine (14) separated from a mother liquor which is an aqueous stream comprising water, ammonia, carbon dioxide, residual melamine and OAT;
said mother liquor (1) is processed including: removal of ammonia (100), producing an ammonia-depleted aqueous stream (4); treatment of said ammonia-depleted aqueous stream in an OAT decomposition section (200) where OAT are decomposed forming a OAT-depleted aqueous stream (6); a purification treatment (300) of said OAT-depleted aqueous stream;
said purification treatment (300) of the OAT-depleted aqueous stream (6) is configured to produce: a vapour stream (7) containing ammonia, CO2 and water; a first liquid stream (8) and a second liquid stream (9);
wherein said first liquid stream (8) contains residual ammonia and CO2, and said first liquid stream has a concentration of CO2 not greater than a target CO2 concentration; said second liquid stream (9) has a concentration of ammonia not greater than a target ammonia concentration,
wherein at least part of said first liquid stream (8) and/or at least part of said second liquid (9) stream is recycled to the melamine production process.

2. A process according to claim 1 wherein: the target CO2 concentration is 500 ppm by weight, preferably 250 ppm by weight, more preferably 150 ppm by weight; and/or wherein the target ammonia concentration is 50 ppm by weight, preferably 25 ppm by weight, more preferably 10 ppm by weight.

3. A process according to claim 1 or 2, wherein at least part of said first liquid stream (8) is recycled to the melamine purification section and is mixed with the raw melamine stream to produce said ammonia solution of melamine.

4. A process according to any of the previous claims wherein the solid melamine (14) effluent from the solid-liquid separation can be washed, preferably with water, and dried with hot air (25), and wherein said hot air is scrubbed (600) with at least part of said second liquid stream (9); and/or wherein at least part of said second liquid stream is recycled as a water make-up to the melamine production process.

5. A process according to any of the previous claims wherein: the removal of ammonia from the aqueous stream is performed in an ammonia removal section (100); said vapour stream (7) obtained from the purification treatment (300) of the OAT-depleted aqueous stream (6) is sent to said ammonia removal section to recover ammonia and CO2 contained therein.

6. A process according to any of the previous claims, wherein said purification (300) of the OAT-depleted aqueous stream (6) is performed in at least one stripping column (301), wherein said first liquid stream (8) is withdrawn as a lateral stream of said stripping column, and said second liquid stream (9) is a bottom stream of said stripping column.

7. A process according to claim 6 wherein the vapour stream (7) effluent from the top of said stripping column (301) is subjected to total condensation in a total condenser and the resulting condensed stream is partly recycled as reflux of said column and in part is sent to a tied-in urea production process or to an offgas condensation step (700) to favor condensation of said offgas stream (11) effluent from the melamine synthesis process (400).

8. A process according to any of the previous claims wherein the purification of the OAT-depleted stream (300) is performed at a pressure greater than the pressure of the ammonia removal from the mother liquor (1).

9. A process according to any of the previous claims wherein the purification (300) of the OAT-depleted stream (6) is operated at a pressure from 2 barg to 7 barg, preferably from 2.5 barg to 5 barg.

10. A process according to any of the previous claims wherein the mother liquor (1) is heated up to a temperature from 70 °C to 95 °C before being treated for ammonia removal (100).

11. A process according to any of the previous claims wherein the ammonia removal (100) is performed in a distillation column (101) provided with a partial condenser (103).

12. A process according to claim 11 wherein an effluent from the top of the distillation column (101) is partially condensed in said partial condenser (103) to form:
an ammonia-rich vapour stream (2) having a concentration of ammonia preferably not lower than 80% by weight, more preferably not lower than 85% by weight, still more preferably not lower than 90%; and a concentration of CO2 not greater than 1% by weight, more preferably not greater than 0.6% by weight;
a liquid stream (3) comprising ammonia, carbon dioxide, and preferably less than 75% by weight of water, more preferably from 40% to 75% by weight, still more preferably from 45% to 70% by weight, further preferably from 50 to 65% by weight.

13. A process according to claim 12 wherein the distillation column (101) and the partial condenser (103) are operated at a pressure such that the liquid stream (3) remains liquid at temperatures reachable with cooling water during partial condensation, preferably said pressure ranges from 0.5 barg to 5.0 barg, more preferably from 1.0 barg to 3.0 barg.

14. A process according to claim 12 or 13 wherein at least 70%, preferably at least 80%, of the ammonia fed in the distillation column (101) is withdrawn from said column in the ammonia-rich vapour stream (2).

15. A process according to any of claims 12 to 14 wherein at least 85%, preferably at least 90%, of the carbon dioxide fed in the distillation column (101) is withdrawn from said column in the liquid stream (3).

16. A process according to any of the previous claims wherein hot steam and/or a reboiler (102) provide heat to the step of ammonia removal (100) from the mother liquor (1).

17. A process according to any of claims 11 to 16 wherein the condenser (103) of the ammonia removal step (100) is operated at a temperature ranging from 50 °C to 70 °C, preferably from 55 °C to 65 °C.

18. A process according to any of the previous claims wherein the OAT decomposition section (200) operates at a temperature of at least 230 °C, preferably at least 250 °C, more preferably at least 270 °C, still more preferably at least 285 °C, and at a pressure greater than the vapour pressure of water at the temperature of operation of said decomposition section (200).

19. A process according to any of the previous claims wherein the OAT decomposition section (200) produces a vapour stream (5) that is sent to any of the following steps or to a combination thereof:
condensation of offgas (700) and recycle (24) to a tied-in urea production process;
an ammonia removal step (100);
a water purification treatment (300).

20. A process according to any of the previous claims wherein the OAT-depleted aqueous stream (6) has a concentration of melamine not higher than 500 ppm by weight, preferably not higher than 100 ppm by weight, more preferably not higher than 20 ppm by weight, and/or wherein the ammonia-depleted aqueous stream (4) has a concentration of ammonia not higher than 5000 ppm by weight, preferably not higher than 2500 ppm by weight, more preferably not higher than 1000 ppm by weight.
